# EUROPEAN PATENT APPLICATION

(11) **EP 2 087 916 A1**
(43) Date of publication of application: **12.08.2009**
(21) Application number: 08101471.4
(22) Date of filing: 11.02.2008
(51) Int. Cl.: A61M 1/16

(54) **Electrosorption device for the purification of blood and other fluids**

(71) Applicant: ICinnovation BV, 5688 RX Oirschot (NL)
(72) Inventor: Simonis, Frank, 5688 RX, Oirschot (NL)
(74) Representative: Verhees, Godefridus Josephus Maria

(57) **Abstract**

The present invention relates to a device for the removal of substances from blood and other fluids, said device comprising i) an electrosorption filter for removing substances such as toxins, toxic solutes, toxic small and middle-sized molecules and protein bound toxins from blood, blood plasma or dialysate fluid, said electrosorption filter comprising a DC power source, a set of electrodes, nanostructured sorption material and/or a porous polymer matrix, ii) an inlet for entry of blood or blood plasma or dialysate fluid into said device, iii) an outlet for the removal of purified blood or blood plasma or dialysate fluid from said device, and iv) a conduit connecting said inlet with said outlet and holding said electrosorption filter such that said blood, blood plasma or dialysate fluid is forced through said electrosorption filter. The device of the present invention is also applicable for the purification of other fluids such as water, chemicals and other biofluids.

## Description

### FIELD OF THE INVENTION

The present invention is in the field of purification of fluids by electrosorption, in particular but not limited to biofluids such as blood, as of use in artificial kidneys, artificial livers and hemodialysis systems. The invention relates to a stand-alone device for the removal of toxic substances from a biofluid, such as from the blood from a patient, to methods of removing toxic substances from blood using the inventive device in a wearable unit enabling continuous blood purification while being mobile. The invention is also applicable to other fluids such as water, process chemicals and other biofluids such as removing substances from urine, dialysate fluids, milk, biochemical analysis and processing fluids. In a reverse mode the invented system can be used to release ingredients in a controlled manner.

### BACKGROUND OF THE INVENTION

Hemodialysis (HD) and peritoneal dialyis (PD) are methods of removing toxic substances (impurities or wastes) from the blood when the kidneys are unable to do so sufficiently. Dialysis is most frequently used for patients who have kidney failure, but may also be used to quickly remove drugs or poisons in acute situations. This technique can be life saving in people with acute or chronic kidney failure. Best known is hemodialysis, which works by circulating the blood along special filters outside the body in a dialysis machine. Here, the blood flows across a semi-permeable membrane (the dialyser or filter), on the other side of which flows a dialysis fluid in a counter-current direction to the blood flow. The dialysing membrane allows passage of substances below a certain molecular cutoff. By diffusion the concentration of these substances will end up being the same on both sides of the membrane. The dialysis fluid removes the toxins from the blood and is generally discarded as waste dialysate. The chemical imbalances and impurities of the blood are being brought back in minimal balance and the blood is then returned to the body. The efficacy of hemodialysis is 10 - 15%, which means that 10 - 15% of the toxins are being removed from the blood. Typically, most patients undergo hemodialysis for three sessions every week. Each session lasts normally 3-4 hours. This is very inconvenient, and the physical and social side effects of dialysis to the patients are a great concern.

In order to provide for portable dialysis devices, that will allow patients to engage in normal daily activities, artificial kidneys have been developed. Essentially there are two types of artificial kidneys.

In one form, the principle of the artificial kidney consists of extracting urea and other more toxic middle molecules from blood by dialysis and regeneration of the dialysate by means of an adsorbent, usually activated carbon. In the case of a system based on such a dialysis kidney machine, a key aspect resides in regenerating the dialysis fluid when the latter is to be recycled into the dialyser. Dialysis kidney machines that can be encountered in the prior art include for instance those described in GB 1 406 133, and US 2003/0097086. GB 1 406 133 discloses an artificial kidney of the recycle type having an improved adsorbent comprising activated carbon and alumina. US 2003/0097086 discloses a portable dialysis device comprising dialyzers connected in series that utilize dialysate, and further comprising a plurality of contoured sorbent devices, which are connected in series and are for regenerating the spent dialysate. As adsorption materials for regeneration of the spent dialysate, activated charcoal, urease, zirconium phosphate, hydrous zirconium oxide and/or activated carbon are provided.

In another form, the principle of the artificial kidney may be based on ultrafiltration, or hemofiltration, using appropriate membranes, wherein large molecules including blood cells are retained in the retentate on the filter, and the toxic substances are collected in the (ultra)filtrate. During hemofiltration, a patient's blood is passed through a set of tubing (a filtration circuit) via a machine to a semipermeable membrane (the filter) where waste products and water are removed. Replacement fluid is added and the blood is returned to the patient. In a similar fashion to dialysis, hemofiltration involves the movement of solutes across a semi-permeable membrane. However, the membrane used in hemofiltration is far more porous than that used in hemodialysis, and no dialysate is used-instead a positive hydrostatic pressure drives water and solutes across the filter membrane where they are drained away as filtrate. An isotonic replacement fluid is added to the resultant filtered blood to replace fluid volume and valuable electrolytes. This is then returned to the patient. Thus, in the case of ultrafiltration, a key aspect resides in separating the urea from the other components in the ultrafiltrate such as salts which have also passed through the membrane but which must be reincorporated into the blood in order to maintain the electrolyte composition thereof substantially constant.

A combination of the two systems described above has also been proposed. Shettigar and Reul (Artif. Organs (1982) 6:17-22), for instance, disclose a system for simultaneous filtration of blood using a hemofilter and dialysis against its purified filtrate, wherein the filtrate is purified by a multi-adsorption system consisting of charcoal for removal of urea and a cation exchanger.

Intermediate systems, i.e. systems that perform no ultrafiltration, yet which adsorb toxic substances directly from the blood have also been proposed. US 2004/0147900 discloses a cartridge for treating medical or biological fluid, in particular blood, consisting of a compartmentalized container, wherein each compartment contains adsorbing particles. The adsorption materials proposed are essentially those disclosed in US 2003/0097086 described above, and thus may effectively remove urea from blood.

As noted above, the adsorbent for regenerating the dialysate is usually activated carbon. However other adsorbents have been proposed for the removal of substances from dialysis fluids or ultrafiltrate. US 3,874,907, for instance, discloses microcapsules consisting essentially of a crosslinked polymer containing sulphonic acid groups and coated with a polymer containing quaternary ammonium groups, for use in an artificial kidney. Examples of the sulphonated polymer include sulphonated styrene/divinyl benzene copolymer and examples of the coating polymer include those obtained by polymerization of for instance vinyldimethylamine monomers. Shimizu *et al.* (Nippon Kagaku Kaishi (1985), (6), 1278-84) described a chemisorbent composition for the removal of urea from dialysis fluid or hemofiltrate for use in an artificial kidney. The chemisorbent is based on dialdehyde starch (DAS)-urease conjugates and 4,4'-diamidinodiphenylmethane (DADPM).

Another approach relates to electrodialysis, a method to fasten the dialysis process by applying an electrical field over the dialysate membrane similar to electrophoresis systems. For instance in WO03020403 a dialysate system is proposed with an electrical voltage over the membrane. The proposed voltage is in the range of 50-150 Volts. It is claimed that the electrical field promotes the diffusion rate and hereby the clearance rate of toxins such as small solutes, phophate, creatinine, beta2microglobuline and even urea. A major drawback however is the required high voltage resulting in significant heating of the blood. This system therefore requires an additional cooling section making the system bulky and energy consuming.

Another relevant approach is known from purification of water and is called electrosorption. E.g. in US2007/00728431 an apparatus is disclosed for removing inorganic ions such as salt and metals from water by means of carbon electrodes that are activated with a small voltage. This system seems to work well for water and inorganic substances. Nothing has been disclosed so far for removing organic molecules and substances such as toxic small and middle molecules and proteins via electrosorption.

In conclusion, the prior art discloses both dialysing and ultrafiltration devices, wherein various substances may be used as sorbents. Also the use of an external electrical field to boost the dialysate diffusion process has been disclosed.

The problem with the system of the prior art is that however, that they are still too large due to limited sorption capacity of the materials, or not efficient or both, in order to allow small, desk-top sized or wearable blood purification systems.

It is an object of the present invention to overcome the problems associated with the devices of the prior art and to provide a compact and efficient sorption-filter system for use in hemodialysis and peritoneal dialysis systems and in a wearable artificial kidney.

### SUMMARY OF THE INVENTION

This problem is solved according to the invention by providing a device comprising:
i) a housing having an inlet for entry of dialysate fluid, blood or blood plasma into said housing, an outlet for the removal of purified dialysate fluid, blood or blood plasma and excess fluid from said housing, and a conduit connecting said inlet with said outlet
ii) an electrosorption filter for removing toxins, toxic solutes, toxic small and middle-sized molecules and protein bound toxins from the dialysate fluid, blood or bloodplasma, said electrosorption filter being contained in said conduit such that said dialysate fluid, blood or blood plasma must pass through said electrosorption filter when flowing from said inlet to said outlet and said electrosorption filter comprising:
   a) a sorption material selected from the group consisting of:
      1) a nanostructured sorption material;
      2) a porous polymer matrix, wherein the pores in said matrix are of a size that allows the entry into said matrix of toxic substances sought to be removed from said liquid and/or preventing the entry into said matrix of substances not sought to be removed from said liquid; and
      3) a nanostructured sorption material captured in a porous polymer matrix, wherein the pores in said porous polymer matrix are of a size that allows the entry into said matrix of substances sought to be removed from said liquid while preventing the escape of said nanostructured sorption material and/or preventing the entry into said matrix of substances not sought to be removed from said liquid; said sorption filter being contained in a housing;
   b) a set of electrodes coated with or in electrical contact with the sorption material;
   c) a power source to provide the electrodes with an electrical charge in order to generate an electrical field strength in the sorption medium.

Said toxic substances are preferably selected from potassium, phosphate, urea, uric acid, ammonia, creatinine, beta2-microglobulin (β2M), and albumin-bound toxins such as paramino hyppuric acid and bilirubin The electrosorption filter combines nanostructured sorbent materials with a high specific and selective surface area together with additional electric surface charging delivered by an external power source. This combination enables a very efficient, highly effective and therefore small sized sorption system.

The small size and subsequent low weight allows for a small, desk-top size system and even a wearable device for blood filtration or hemodialysis or peritoneal dialysis is feasible.

Said electrosorption filter comprises an absorbing, adsorption, ion-exchange and/or surface crystallisation material with very small nano sized particles or pores offering a large specific surface area in combination with a high chemical surface activity like activated carbon, nanoclays, nanocrystalline hydrotalcites, nanoporous silica's, nanoporous or layered alumina silicates (like zeolites), nanoporous metal oxides and metal hydroxides, metal organic frameworks, zeolite imidazolate frameworks, nanosized and /or activated graphite, cyclo-dextrines, crystallisation seeds, a highly porous matrix material with again a large specific surface area, with tunable porosity and a high specific chemical activity like carboxymethyl cellulose and like a biopolymer such as oxidized starch modified with functional groups for specific absorption or combinations thereof.

An embodiment of the device according to the invention is characterized in that the nanostructured sorption material is selected from the group consisting of:
i) nanoparticles or nanocrystalline materials, preferably metal silicates such as nanoclays, preferably an exfoliated nanoclay, metal hydroxides, preferably layered double hydroxides like a nano-hydrotalcite or pure metaloxide nanoparticles;
ii) nanoporous materials, preferable selected from zeolites, mesoporous systems, carbonaceous nanomaterials and metal organic frameworks;
iii) nanocomposites;
iv) nanofibers, and
v) any combination of the above.

In another preferred embodiment of a device of the present invention said porous polymer matrix is based on a cross-linked polymer and/or a charged polymer and/or a polymer modified with specific functional groups for specific absorption.

In yet another preferred embodiment said polymer is a biopolymer selected from carbohydrates and proteins.

In still another preferred embodiment said carbohydrate is an oxidized crosslinked starch.

In still another preferred embodiment said carbohydrate is a carboxymethyl cellulose.

In another preferred embodiment the sorption material is provided in the form of a permanent coating on the electrode, a replaceble gel or replaceble dried granules having a mean size over the range 250 microns to 1500 microns based on the size in dried form.

Preferably the pores in said matrix are of a size that prevents the entry into said matrix of albumin and other useful blood components such as transferrin and vitamin B 12.

A further embodiment of the device according to the invention is characterized in that said electrosorption filter is provided in the form of a cartridge with said electrodes coated with said sorption material or with said electrodes surrounded by a porous envelop containing said sorption material.

Another embodiment of the device according to the invention is characterized in that said electrosorption filter is combined with a permeable envelop to form a filter pad comprising an envelop surrounding a filter pad contents, wherein the envelop of said pad comprises a permeable membrane and the contents of said pad comprise said sorption material.

A further embodiment of the device according to the invention is characterized in that said device further comprises a hemofilter for separating patient blood plasma from patient blood cells.

In this embodiment said sorption-filter is combined with a hemofilter system in order to form an artifical kidney device for the removal of toxic substances from the blood from a patient, comprising a hemofilter for separating patient blood plasma from patient blood cells, electrodes in combination with a sorption-filter for removing toxins, toxic small- and middle-sized molecules and protein bound toxins from the patient blood plasma and optionally to supplement the blood with at least one substance selected from the group consisting of vitamins, minerals, anticoagulants, anti microbial agents and other medicaments.

In case of blood filtration, a hemofilter for separating patient's blood plasma from the blood is used followed by the electrosorption filter for removing toxic ionic solutes, toxic small- and middle-sized molecules and protein bound toxins from the patient's blood plasma.

In yet another preferred embodiment said device is part of a hemodialysis or peritoneal dialysis system in order to improve the absorption capacity of the dialysis fluid, to mimimize the dialysate fluid volume and/or to minimize the dimensions of the dialysate system, thereby allowing the dialysate system to be wearable.

In yet another preferred embodiment said dialysis system is a wearable dialysis system.

In a further preferred embodiment said hemofilter comprises at least a further outlet for recovery of patient blood cells.

Preferably said hemofilter and said electrosorption filter are combined to form the filter pad wherein said permeable membrane is formed by said hemofilter.

A still further embodiment of the device according to the invention is characterized in that said device further comprises an electrosorption filter for removing toxins, small and middle-sized molecules from the patient blood plasma.

Yet a further embodiment of the device according to the invention is characterized in that said device further comprises means for supplementing said dialysate fluid and/or said (purified) blood plasma with at least one substance selected from the group consisting of vitamins, minerals, anticoagulants, anti microbial agents and other medicaments.

Preferably said device further comprises ion exchange systems and materials in combination with the electrical charge from the electrodes.

A further embodiment of the device according to the invention is characterized in that the performance of the sorption filter is being monitored with a sensor system measuring the quality of the dialysate, blood or bloodplasma in the outlet.

A still further embodiment of the device according to the invention is characterized in that the device comprises a control unit controlling the sorption activity of the sorption material by a voltage between 0-200V of an external power source in order ensure a field strength in the sorption medium of typically 10-20 V/cm.

In order to enhance and to regulate the sorption capacity, the sorption material is connected via electrodes to an external DC electrical power source such as a battery or a rectifier. Hereby, the electrical surface charge of sorption material can be externally controlled. A voltage in the range of 0-3 V, with typical values of 0,5-1,5 V is used. A higher voltage will increase the chance on electrolysis of the water with negative effects on the sorption capacity. By regulating the voltage and electrical current of the power source, the surface charge of the sorption material can be augmented, maintained, diminished or reversed. Since the surface charge is the major driving force for molecular adsorption and binding, the capacity of the sorption material can be increased or decreased depending on the need. The sorption material can also be regenerated by operating the device in reversed mode, with a voltage reversal on the electrodes leading to a repulsion of bound toxins. As a side effect, the local electrical field will promote the diffusion of the charged toxic molecules into the sorption materials by some extent.

Preferably the voltage of the external source is electronically regulated depending on the reading of the sensor system.

The electrodes are preferably planar, circular, tubular or thread electrodes made from Ag, Ti, stainless steel, Cr or other blood compatible materials or with a coating hereof.

In a preferred embodiment said artificial kidney system is a system that improves the clearance performance of existing hemodialysis and peritoneal systems.

In a preferred embodiment said artificial kidney system is a system that eliminates the use of a dialysate fluid in an existing hemodialysis system.

In a preferred embodiment said artificial kidney system is a wearable device.

The present invention provides a device for the removal of toxic substances from a biofluid such as blood, blood plasma or dialysate, said device comprising:
i)a compartment with a set-up of electrodes and sorption materials allowing extraction of toxins from the fluid in the compartment by electrosorption and also desorption of toxins by reversed electrical operation,
ii)electrodes that are coated with a sorption material or electrodes that are in electrical contact with sorption materials in the said compartment, allowing the sorption material to be activated or de-activated by the electrical charge delivered by the electrodes and the external power source,
iii)a sorption material for removing toxins, small and middle-sized molecules from the biofluids, said sorption filter comprising a sorption material selected from the group consisting of:
   - a nanostructured sorption material offering a high specific surface area with specific (ab/ad)sorption affinity;
   - a porous polymer matrix, wherein the pores in said matrix are of a size that allows the entry into said matrix of toxic substances sought to be removed from said liquid and/or preventing the entry into said matrix of substances not sought to be removed from said liquid; and
   - a nanostructured sorption material captured in a porous polymer matrix, wherein the pores in said porous polymer matrix are of a size that allows the entry into said matrix of substances sought to be removed from said liquid while preventing the escape of said nanostructured sorption material and/or preventing the entry into said matrix of substances not sought to be removed from said liquid.
iv) an inlet for entry of the biofluid into said device,
v) an outlet for the removal of purified biofluid from the said device, and
vi) a conduit connecting said inlet with said outlet and holding a compartment with said electrodes and said sorption filter such that the biofluids must pass through said compartment with electrodes and sorption material when flowing from said inlet to said outlet.

In a preferred embodiment of a device of the present invention said device comprises a compartment with:
a) planar, circular, tubular or thread electrodes made from Ag, Ti, stainless steel, Cr or other blood compatible materials or with a coating hereof,
b) said electrodes being coated with or in electrical contact with a porous, preferably nanostructured sorption material,
c) an electrical control unit capable of controlling the electrical voltage on each of the electrodes,
d) a sensor unit capable of measuring the toxin concentration in the cleaned biofluids, e.g. via an electrical conductivity measurement,
e) a pumping unit to ensure sufficient biofluid flow through the compartment, in case of bloodplasma of a renal patient this is typically 30 ml/min,
f) optional filters such a hemofilter for blood - blood plasma separation

### BRIEF DESCRIPTION OF THE DRAWINGS

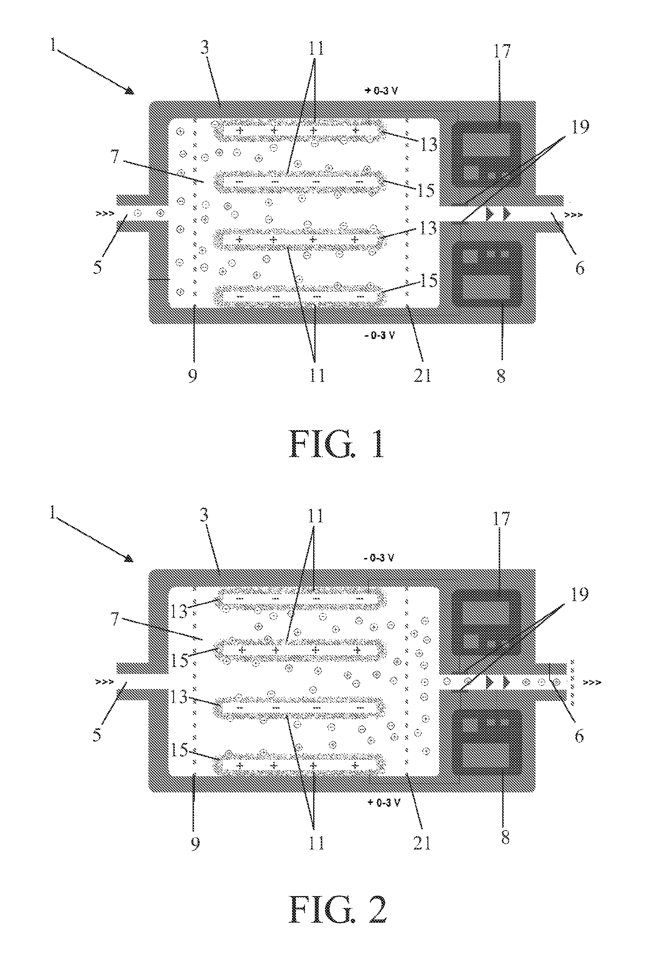

Figure 1 shows a cross-sectional presentation of a device according to the invention with electrodes surrounded with an envelop filled with nanostructured sorption materials. The device is in the clearance mode. Toxins are being entrapped by a combined effect of the chemical affinity of the sorption materials for the toxins and the electrical surface charge provided by the electrodes.
Figure 2 shows a cross-sectional presentation of a device according to the invention with electrodes surrounded by an envelop filled with nanostructured sorption materials. The device is in the regeneration mode. Toxins are being repelled and released from the sorption material by applying a reversed voltage and subsequent reversed surface charge on the sorption material.

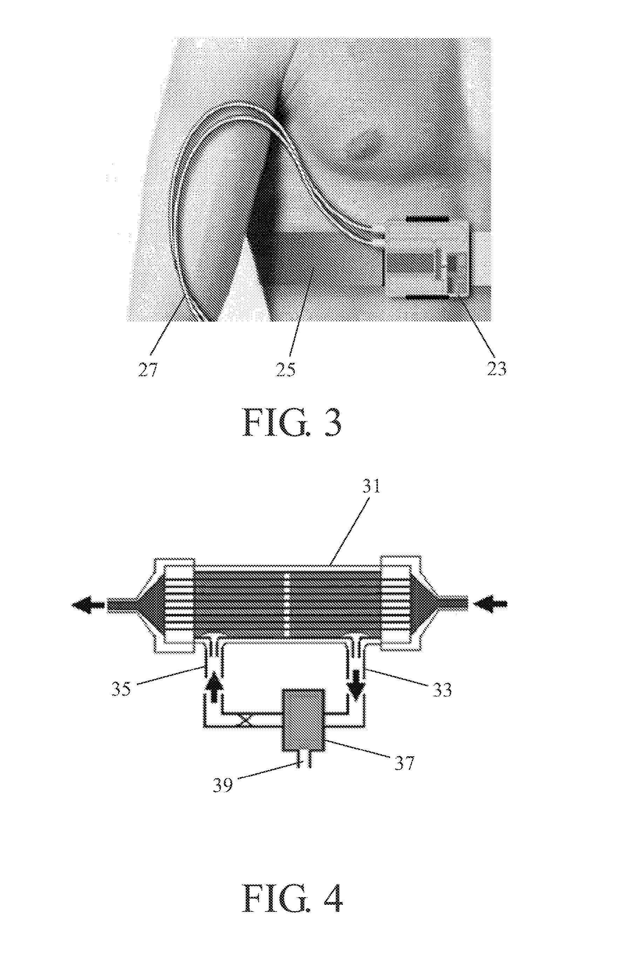

Figure 3 shows an artist impression of wearable device incorporating an electrosorption filer system. Thanks to the nanostructured sorption materials in combination with the electro-activation provided by power sources, a ver small device becomes feasible.
Figure 4 shows a set-up for an existing hemodialysis machine. Here an electrosorption filter is used in combination with a traditional hemofilter. This allows hemodialysis without the use of a dialysate, saving 120 L dialyate per treatment. Thanks to the nanostructured sorption materials and the electro-activation, a much better clearance of the blood is achieved especially for the toxic middle molecules and protein bound toxins.

### DETAILED DESCRIPTION OF THE INVENTION

The term "sorption" as used herein, refers to both adsorption and absorption. Adsorption is a process that occurs when a gas or liquid or solute (called adsorbate) accumulates on the surface of a solid or more rarely a liquid (adsorbent), forming a molecular or atomic film (adsorbate). It is different from absorption, where a substance diffuses into a liquid or solid to form a "solution". The term sorption encompasses both processes, while desorption is the reverse process.

The term " small-sized molecules", as used herein, refers to molecules with a molecular weight lower than 500 Da, such as uric acid, urea, guanidine, ADMA, creatinine.

The term " middle-sized molecules", as used herein, refers to molecules with a molecular weight between 500 Da and 5000 Da, such as end products from peptides and lipids, amines, amino acids, protein bound compounds, cytokines, leptins, microglobulins and some hormones.

The term "nanoporous materials" refers to materials having pores that are by definition roughly in the nanometre range, that is between 1x10⁻⁷ and 0.2x10⁻⁹ m and includes reference to the 3 categories set out by IUPAC of microporous materials (such as zeolites) having pore sizes of 0.2-2nm; mesoporous materials having pore sizes of 2-50nm; and macroporous materials having pore sizes of 50-1000nm.

The term "ionic solutes", as used herein, refers to components such as phosphates, sulphates, carbon hydrates, chlorides, ammonia, potassium, calcium, sodium.

"Nano sized" as used herein, refers to a size of approximately 1-1000 nm, more preferably 1-100 nm.

The term "electrosorption" refers to a process where ionic solutes and charged molecules in a solution are being adsorpted onto the surface of a sorbent with the help of an additional electrical surface charge on the sorbent provided by an electrical power source.

The term "electrosorption filter" refers to a filter system comprising a sorbent that can be electrically charged or discharged with an external power source via electrodes connected to the sorbent material.

### The purification device holding an electro-sorption-filter package

A device of the present invention can take the form of an electrosorption filter package that is placed in the dialysis fluid system of a hemodialysis or peritoneal dialysis system, enabling the removal of toxins from the dialysis fluid. The electrosorption filter continuously purifies the dialysate fluid, keeping the the toxin concentration in the dialysis fluid low, resulting in an improvement of the hemodialysis and peritoneal dialysis efficiency, typically with 100%, and reduces the consumption of dialysis fluid needed dramatically, ideally down to 1-10 litres. An additional and optional function of the sorption filter is to release ingredients for supplementing of the blood such as calcium, vitamin A, C and B12, anti-coagulation agents, anti microbial agents, minerals, specific medicaments etc. This option will simplify the operation of existing hemodialysis and peritoneal dialysis systems and will reduce the chance on occurring infections in the peritoneal dialysis system.

A device of the present invention can take the form of wearable peritoneal dialysis system wherein the electrosorption filter package is placed in a wearable peritoneal dialysis system. Due to the continuous filtering of the electrosorption filter, the volume of dialysate fluid can be reduced to typically 1-2 litres. The wearable peritoneal dialysis device comprises a tubular access system to the abdominal cavity and a unit comprising a fluid pump, power, sensors, electronic control, a facility to place and replace said electrosorption filter package, typically on a daily basis and a system to dispose off excess water. An additional and optional function of the sorption filter is to release ingredients for supplementing the blood, such as calcium, vitamin A, anti-coagulation agents, anti microbial agents, minerals, specific medicaments etc. This option will enhance the operation of the peritoneal dialysis system and will reduce the chance on occurring infections.

A device of the present invention can take the form of wearable hemodialysis system wherein the electrosorption filter system is placed in a wearable hemodialysis system. Thanks to the continuous filtering of the electro-sorption filter, the volume of dialysate fluid can be reduced to typically 1-2 litres. The wearable hemodialysis device comprises a vascular access tubing system and a unit comprising a small hemofilter system, fluid pump, power, sensors, electronic control, a facility to place and replace said electrosorption filter package, typically on a daily-to-weekly basis, and a system to dispose off excess water. An additional and optional function of the electrosorption filter is to release ingredients for supplementing the blood such as calcium, vitamin A, anti-coagulation agents, anti microbial agents, minerals, specific medicaments etc. This option will simplify the operation of the hemodialysis system and will reduce the chance on occurring infections.

A device of the present invention can take the form of a wearable or desktop sized artificial kidney based on combined blood plasma fitration and electrosorption filtering. In such an embodiment, the blood plasma filtration step will performed by a special high flux hemofilter, with a relative large pore size, that separates blood from blood plasma, allowing toxic solutes, small/middle molecules and albumin with bound toxins to pass with the blood plasma into the compartment with the electrosorption filter package for cleansing. Via an additional hemofilter with a smaller pore size, excess water can be removed from the blood plasma preventing loss of albumin. The cleansed blood plasma is then re-entered into the bloodstream. It will be understood that in such an embodiment, the device further comprises the necessary tubing, vascular access and feedback systems, pumping, electronics, sensors, power packs and other requirements. However, these are not essential to the present invention. An advantage of the artificial kidney device is that no dialysis fluid will be needed. In a preferred embodiment of this device, the hemofilter and electro-sorption-filter are being combined to form a filter package, said filter package comprising an envelop, made from a hemofilter material, surrounding a filter material and electrodes. A representative embodiment of an artificial kidney device of the present invention is depicted in Figures 1 and 2.

The device 1 as depicted in Fig. 1 is a cross-sectional presentation of a device for blood purification in the cleansing mode. The device comprises a housing 3 with an inlet 5 for blood, an outlet 6 for cleansed blood plasma to be returned to the patient, a conduit 7 connecting said inlet with said outlet and a pump 8 for pumping said dialysate fluid, blood or plasma from said inlet to said outlet. The incoming blood with toxins first passes a hemofilter 9 for blood plasma separation. The toxins are then removed from the blood plasma by the combined action of the sorption material 11 and electrodes 13, 15. The electrodes have a coating of nanostructured sorption materials or are in direct contact with a surrounding of nanostructured sorption materials in said compartment. The surrounding sorption material can be in the form of grains or preferably a gel. The positively charged electrodes, with an electrical charge of 0-200 Volt, will attract and bind the negatively charged toxins such as phosphates, creatinine, beta2microglobulin and most of the other toxic proteins. The negatively charged counter electrodes 15 will attract and bind the positively charges toxins such as potassium, ammonia and urea. The electrical driving force for the attraction is the electrical field strength in the sorption medium. A field strength of typically 10-20 V/cm has proven to be quite effective in accelerating the toxins and in promoting the binding. The actual voltage to be applied on the electrodes will therefore depend on the electrode distance. In order to prevent hydrolysis of the fluid, a low voltage is recommend, typically 1.2-1.4 V. Such a low voltage is only effective if the electrode distance is small, typically in the range of 0.5-1.0 mm. However, if hydrolysis or other side effects of a high voltage in the fluid can be accepted, an electrode distance of several cm's is possible.

The nanostructured sorption materials offer a very large surface area and chemical activity to bind a high load of toxins, typically in the range of 10-50% on weight basis. This purification mode can be operated until the sorption material is saturated with toxins. This depends on the amount of sorbent used. A representative embodiment will comprise 20-40 grams of sorbent, allowing a continuous purification mode to last 12-24 hours. The control unit 17 with sensors 19 in Fig. 1 will give a signal when the sorbent has reached its maximum capacity. Then the device will be turned into the reverse mode as depicted in Fig. 2.

In Fig 2, the device is set into reverse mode by switching the external voltage into its opposite sign. With the reversed voltage, the sorption function changes into a repellent function and the toxins are enforced to unbind and to leave sorbent surface. Hereby the sorbent is being cleaned and regenerated. The released toxins in the compartment are removed by disposing the fluid. Before disposal, the fluid is filtered over an additional hemofilter 21 to prevent albumin loss. The volume of the disposed fluid is typically around 0,5-1 L per day, the normal amount of excess fluid to be released by a human.

In Fig 3 an artist impression is shown of the wearable artficial kidney 23, held by a belt 25 and connected to the blood system of the patient by two blood lines 27. These blood lines will be attached to the vanes the just below the neck of the patient.

The set-up as depicted in Fig. 4 shows the electrosorption filter package in combination with a traditional hemofilter system. The hemofilter 31 for separating blood and blood plasma is here external and connected with fluid connectors 33 and 35 to the electrosorption filter 37 with its own outlet 39 for excess fluid and toxin release. Such a system can be used in traditional hemodialysis equipment to replace the dialysate fluid and to improve the clearance, especially in terms of clearing middle molecules and albumin.

The blood (or general fluid) inlet 5 may be connected to vascular access and tubing. The blood plasma (or general purified fluid) outlet 7 is provided for recovery of the filtered blood plasma. The sensor 19 is for measuring salt and/or small and/or middle-sized molecule content, etc.; The control unit 17 comprising electronic components (e.g. for digital signal processing (DSP), interfaces for sensors); a RF-wireless radio module providing a link to a medical consultant or computer; a power supply for the device (e.g. incl. power supply for the pump) and accompanying electronics and mechatronic part for the pump. The housing 3, optionally comprised of a monolithic structure. The housing is suitably injection moulded or otherwise formed plastic housing parts. The outlet system to dispose off of excess water may take the shape of a simple drain (valve under the filter package) connected to a tube, container, a fluid absorbing package etc. It will be understood that the device of the present invention may take various shapes and the various components may take various positions relative to each other. In fact, the skilled person is free in the design of the device and the location of the various components as long as functionality will be maintained.

The hemofilter in a device of the present invention is preferably a commercially available hemofilter (e.g. such as produced by Gambro GmbH, Hechingen, Germany or Membrana GmbH, Wuppertal, Germany).

In an alternative embodiment the electrosorption filter in a device of the invention may comprise an inlet for receiving patient blood plasma exiting said hemofilter, and at least one outlet for recovery of purified blood plasma.

A device of the present invention may, in any embodiment, further comprise means for supplementing the (purified) blood plasma or dialysate fluid with at least one substance selected from the group consisting of vitamins such as vitamins A,C and B12; minerals such as calcium, sodium and potassium; anticoagulants; anti microbial agents and other medicaments.

A device of the present invention may, in any embodiment, further comprise means for selective sorption of middle molecules, vitamins and minerals such as calcium, sodium and potassium. The sorption materials as described (smectites, nanoclay, layered double hydroxides, hydrotalcites, crystallisation seeds, metal organic frameworks, modified biopolymers etc.) are therefore loaded with a certain amount of minerals, vitamins and can only absorb a designated amount.

A device of the present invention may, in any embodiment, further comprise means for selective sorption of middle molecules, vitamins and minerals such as calcium, sodium and potassium in the sorption pad via osmotic differences between blood and dialysate fluid in combination with the filterpad. The blood plasma in this variant essentially remains in circulation and does not enter as a whole the filter pad, but is being cleaned from toxic substances and is being supplemented with nutrients and other vital substances via the dialysate fluid in combination with the sorption unit.

Optionally, the device or filter pad may comprise ion exchange systems.

In another aspect, the present invention provides a method for removing toxic substances from blood, comprising using a device according to the present invention.

In another aspect, the present invention provides a method for removing toxic substances from hemodialysis or peritoneal dialysis fluids, comprising using a device according to the present invention.

In another aspect, the present invention provides a method for removing substances from other fluids such as water, e.g. for making drinking water, and for purifying chemicals used in industrial processes as wel as removing substances from other biofluids such as urine, milk, bio-analytical fluids, comprising using a device according to the present invention.

The electrosorption filter in a device of the present invention may take the form of a filter pad, consisting of a rigid or flexible container comprising the sorption materials and electrodes.

The electrosorption filter in a device of the present invention may take the form of a filter cartridge, consisting of a rigid or flexible container comprising a set of built-in electrodes and a replaceble filter pad with sorption materials.

This, in another aspect, the present invention may take the form of a device with built-in electrodes connected to a power supply such as a battery or a rectifier, holding an electrosorption filter cartridge in contact with these electrodes and comprising a blood plasma separator and a sorption filter pad with sorption materials for extracting ionic solutes and small and middel sized molecules from the blood plasma.

### The nanostructured sorption material comprised in the electrosorption filter

The nanostructured material exhibits sorption capacity of various substances, based on ion-exchange, surface adsorption activity and/or surface nucleation (surface crystallisation).

The sorption material is preferably functionalized, such as to exhibit improved sorbing properties of toxic substances such as urea as compared to the non-functionalized material. The sorption material in the present invention is a nanomaterial, meaning that the material contains particles or contains pores with a size of preferably 100 nanometres or less in order create a large specific surface area.

A suitable nanostructured sorption material will be based on a blend of nanomaterial components, each component offering a specific functionality. Depending on the individual needs, a selection is made out of the following nanomaterial classes and components:
i) Layered double hydroxides (LDH's) or so called anionic clays or hydrotalcite-like compounds, comprising an unusual class of layered materials with positively charged layers and charge balancing anions located in the interlayer region. Hydrotalcite-like compounds (HT) can be represented by the following formula: [Mg₁₋ₓ Alₓ (OH)₂]^{x+} [A_{x/n}ⁿ⁻. mH₂0]^{x-}, wherein 0 ≤ x < 0.33, and Aⁿ⁻ is an exchangeable anion having a valence of n. These compounds are similar to the mineral hydrotalcite, Mg₆Al₂(OH)₁₆CO₃.4H₂0. LDH's may have different cations, such as Mg, Mn, Fe, Co, Ni, Cu and/or Zn as divalent cations, and Al, Mn, Fe, Co, Ni, Cr and/or Ga as trivalent cations. Particularly preferred hydrotalcites are Mg₂Fe(OH)₆.OH and Mg.OH. It should be noted that hydrotalcites used in aspects of the present invention need not be layered, but may be provided in the form of nanocrystalline materials.
ii) Nanoclays, phyllosilicates, or layered silicates have negatively charged layers and cations in the interlayer spaces offering ion-exchange, adsorption and surface nucleating activity. Preferred are smectite-like clay minerals, such as montmorillonite, saponite, hectorite, fluorohectorite, beidellite, nontronite, vermiculite, halloysite and stevensite. Very suitable are clays based on magnesium and/or alumina silicate layers with a cation exchange capacity between 50 and 200 milliequivalents per 100 gram. Exemplary nanoclays are available from e.g. Nanocore (USA) and Sudchemie (Germany)
iii) Nanocrystalline metal oxides and metal hydroxides of Mg, Sr, Ba, Ca, Ti, Zr, Fe, V, Cr, Co, Y, Mn, Ni, Cu, Al, Si, Zn, Ag, Au, Mo, Sb, Ce and mixtures thereof e.g. known from WO 2007/051145. The nanocrystalline materials preferably present crystallite sizes of less than about 25 nm, more preferably less than 20 nm, and most preferably less than 10 nm with a surface area of at least about 100 m²/g, more preferably at least about 300 m²/g, and most preferably from about 700 m²/g and more. Exemplary nanocrystalline materials are available from NanoScale Materials, Inc., Manhattan, Kansas, under the name NanoActive®.
iv) Nanoporous materials, characterized by the molecular assembly of structures consisting of nanometer-sized cavities or pores. Nanoporous materials for use in the present invention may include active carbon, nanoporous silica's, nanoporous alumina silicates such as zeolites. Very suitable nanoporous materials for use in the present invention are metal organic frameworks (MOFs). Metal organic frameworks are hybrid materials where metal ions or small nano-clusters are linked into one-, two- or three-dimensional structures by multi-functional organic linkers as described, for example, in US 5,648,508 and US 6,893,564. In many cases it is possible to obtain open micro- and mesoporous structures having high porosities and specific surface areas of even above 5000 m2/g. Such open, porous, structures are very suitable for use as nanostructured adsorbents in the present invention. The metal organic frameworks for use in the present invention may be based on Mg, Sr, Ba, Ca, Ti, Zr, Fe, V, Cr, Co, Y, Mn, Ni, Cu, Al, Si, Zn, Ag, Au, Mo, Sb, Ce or any other metal that provides good adsorption characteristics. The preferred metals in applications for dialysate fluid or blood (plasma) purification in nanostructured materials used in aspects of the present invention are preferably based on metals such as Fe, Ti and Mg. Tectosilicates and tetrasilicates are another class of synthetic or natural aluminosilicates that are crystalline porous nanostructures having long-range crystalline order with pore sizes that may be varied from about 2 Å to 200 Å (Angstroms).
v) Carbonaceous nanomaterials, suitable for use in aspects of the present invention include fullerenes, carbon nanoparticles, diamondoids, porous carbons, graphites, microporous hollow carbon fibers, single-walled nanotubes and multi-walled nanotubes.

A sorbent of the present invention, comprising a nanostructured sorption material captured in a porous polymer matrix, may be prepared by providing the nanostructured sorption material and the porous polymer matrix separately and combining the two such that the nanostructured sorption material is inserted in the porous polymer matrix. This may for instance occur by instilling the polymer matrix with a suspension of the nanostructured material.

Alternatively, in order to obtain a polymer matrix having a nanostructured material dispersed therein, one may prepare the matrix having the desired porosity, and synthesize the nanostructured material therein. Very suitable the polymer matrix may be imbibed with a solution of a metal salt which may then be precipitated the metal salt in the form of a metal hydroxide or metal oxide in the matrix.

### The polymer matrix in the electrosorption filter

A porous polymer matrix is used in order to immobilize the nanostructured sorption material and to prevent unwanted leakage of nanomaterials. The porous structure of the polymer matrix will allow for the trapping of molecules of which the removal from the fluid is sought such as the ionic solutes, small and middle molecules such as urea, creatinine and billirubin.

In other to provide for a matrix having a specific pore size, the polymer in preferred embodiments is a cross-linked polymer. The porosity of the polymer matrix is crucial for enabling selectivity: a high diffusion rate towards the sorption sites is sought for the toxins to be removed, while specific blood components such as albumin need to be prevented for sorption. Since an albumin molecule is approximately an 80 Angstrom diameter sphere, a suitable pore size for the polymer matrix in applications for blood (plasma) or dialysate purification would be less than 80 Angstrom (less than 8 nm).

In a preferred embodiment the polymer with the nanostructured sorption materials can be electrically surface charged via electrodes. In another preferred embodiments the polymer is a chemically charged polymer.

Polymers used in aspects of the invention may be synthetic or natural polymers. Natural polymers (biopolymers) suitably comprise a cross-linked carbohydrate or protein, made of oligomeric and polymeric carbohydrates or proteins. The biopolymer is preferably a polysaccharide. Among these, the "starch family", including amylose, amylopectin and dextrins, is especially preferred. Other suitable polysaccharides include glucans and celllulose. A preferred cellulose is carboxymethylcellulose (CMC, e.g. AKUCELL from AKZO Nobel).

Carbohydrates which can thus be used are carbohydrates consisting only of C, H and O atoms. Preferably, oligomeric carbohydrates with a degree of polymerization (DP) from DP2 on or polymeric carbohydrates from DP50 on are used. Most preferably the oxidized starch is crosslinked. A preferred crosslinking agent is di-epoxide. Very suitably, the crosslinking level is between 0.1 and 25%, more preferably between 1 and 5%, and most preferably between 2.5 and 3.5%.

Proteins which can be used include albumin, ovalbumin, casein, myosin, actin, globulin, hemin, hemoglobin, myoglobin, gelatin and small peptides. In the case of proteins, proteins obtained from hydrolysates of vegetable or animal material can also be used. Particularly preferred protein polymers are gelatin or a derivative of gelatin.

Also suitable mixtures of carbohydrates (e.g. copolymers) or mixtures of proteins can be used.

In order to provide for a charged polymer, the carbohydrate polymer may for instance be modified by oxidation, substitution with cationic functional groups or with carboxymethyl groups, or by esterification with e.g. acetyl groups. Particularly preferred carbohydrate polymers are chosen from the group consisting of starch or a derivative of starch, cellulose or a derivative of cellulose, pectin or a derivative of pectin.

Modification of the polymers can be accomplished by oxidation, substitution with cationic functional groups or carbonyl and/or carboxymethyl groups and/or esterifying with e.g. acetyl groups. Although in the latter case no charge is added, it is used to make the polymer more hydrophobic to allow complexing of the polymer with nanostructured sorption materials that have little or no charge.

Generally the polymers will be modified before cross-linking and gelation. It is possible to modify the polymer after cross-linking and gelation only if cross-linking is performed by ether-formation. The person skilled in the art will know how to modify the polymers specified in the invention to provide them with the mentioned groups.

The charge of the cross-linked polymer can be negative or positive depending on the type of polymer, the type of modification and the type of cross-linking. Advantageously, the polymers are of considerable size, i.e. 30 kD or more. This allows for the ready formation of a gel upon cross-linking and it allows for the formation of a lattice, which is capable of taking up the nanostructured sorption material.

The selectivity of the sorption material of the present invention can further be enhanced by loading the material with specific molecule catchers or receptors receptors such as antibodies, prosthetic groups or carboxyl groups (general: binding partners). For selective binding of proteins or degenerated proteins, antibodies are very suitable, e.g. from the immunoglobulin superfamily (IgSF), prosthetic groups e.g. from lipid and vitamin derivatives or metal ion such as gold, iron, zinc, magnesium, calcium that covalently bond to proteins, as well as carboxyl groups that can bind to proteins by forming peptide bonds. Such selective absorbing agents can further enhance the selectivity of the filter pad for toxic proteins.

In a preferred embodiment, the device of the present invention comprises means for regulating the content of minerals and other substances in the blood plasma via selective sorption and controlled release.

In a preferred embodiment therefore, the device for the removal of toxic substances from blood from a patient, comprises a hemofilter and an electrosorption filter, wherein the electrosorption filter removes toxic solutes, small and middle-sized molecules from the blood based, and includes such functions as selective sorption, controlled release and antimicrobial control.

In a most preferred embodiment, the hemofilter, and electrosorption filter are separate parts. For instance, the hemofilter may consist of an existing, commercially available albufilter and the electrosorption filter may consist of a cartridge with built in electrodes. In this cartridge also the sorption material is contained. The sorption material can be held in a porous envelop to form a filter pad. The general design of such pads is well known in coffee-machines, such as the Senseo^{®} system. This type of filtering pad (pill-shaped pouch) will allow simple inclusion of materials for (slow) release of additional components or additional (an)ion exchange systems. In this form it is referred to herein as a sorption and release system.

It will be understood that the advantage of such a filter pad is that it forms a disposable and replacement part of the device, and can be replaced by a fresh filter pad, for instance when it has been saturated with toxic substances, or if one or more of the components supplemented to the plasma have run out. The sorption capacity of the filter pad for urea and the middle molecules is typically in the range of 50-100% of its own weight.

The device of the present invention can be used for filtering or purification of blood of patients with a (developing) renal failure. In a preferred embodiment, the device takes the form of a wearable artificial kidney device, but can also be embodied in desktop sized equipment or in adapted hemodialysis or peritoneal dialysis equipment.

The artificial kidney is able to perform some of the functions which normally will be done by a properly functioning human or animal kidney, in particular filtering of blood and regulation and control of the content of substances in the blood.

## Claims

1. A device for the removal of substances from fluid, moreover substances from biofluid in particular toxic substances from dialysate fluid, blood or blood plasma, **characterized in that** said device comprising:
i) a housing having an inlet for entry of dialysate fluid, blood or blood plasma into said housing, an outlet for the removal of purified dialysate fluid, blood or blood plasma and excess fluid from said housing, and a conduit connecting said inlet with said outlet
ii) an electrosorption filter for removing toxins, toxic solutes, toxic small and middle-sized molecules and protein bound toxins from the dialysate fluid, blood or bloodplasma, said electrosorption filter being contained in said conduit such that said dialysate fluid, blood or blood plasma must pass through said electrosorption filter when flowing from said inlet to said outlet and said electrosorption filter comprising:
a) a sorption material selected from the group consisting of:
1) a nanostructured sorption material;
2) a porous polymer matrix, wherein the pores in said matrix are of a size that allows the entry into said matrix of toxic substances sought to be removed from said liquid and/or preventing the entry into said matrix of substances not sought to be removed from said liquid; and
3) a nanostructured sorption material captured in a porous polymer matrix, wherein the pores in said porous polymer matrix are of a size that allows the entry into said matrix of substances sought to be removed from said liquid while preventing the escape of said nanostructured sorption material and/or preventing the entry into said matrix of substances not sought to be removed from said liquid; said sorption filter being contained in a housing;
b) a set of electrodes coated with or in electrical contact with the sorption material;
c) a power source to provide the electrodes with an electrical charge in order to generate an electrical field strength in the sorption medium.

2. Device according to claim 1, **characterized in that** the nanostructured sorption material is selected from the group consisting of:
i) nanoparticles or nanocrystalline materials, preferably metal silicates such as nanoclays, preferably an exfoliated nanoclay, metal hydroxides, preferably layered double hydroxides like a nano-hydrotalcite or pure metaloxide nanoparticles;
ii) nanoporous materials, preferable selected from zeolites, mesoporous systems, carbonaceous nanomaterials and metal organic frameworks;
iii) nanocomposites;
iv) nanofibers, and
v) any combination of the above.

3. Device according to claim 1 or 2, **characterized in that** said nanostructured sorption material captured in a porous polymer matrix is based on a cross-linked polymer and/or a charged polymer, preferably a biopolymer selected from selected from carbohydrates and proteins, wherein said carbohydrate is selected form an oxidized crosslinked starch and a carboxymethyl cellulose.

4. Device according to claim 2 or 3, **characterized in that** the pores in said matrix are of a size that prevents the entry into said matrix of albumin.

5. Device according to claims 2, 3 or 4, **characterized in that** said electrosorption filter is provided in the form of a cartridge with said electrodes coated with said sorption material or with said electrodes surrounded by a porous envelop containing said sorption material.

6. Device according to claim 2, 3 or 4, **characterized in that** said electrosorption filter is combined with a permeable envelop to form a filter pad comprising an envelop surrounding a filter pad contents, wherein the envelop of said pad comprises a permeable membrane and the contents of said pad comprise said sorption material.

7. Device according to any one of the preceding claims, **characterized in that** said device further comprises a hemofilter for separating patient blood plasma from patient blood cells.

8. Device according to any of the preceding claims, **characterized in that** said device further comprises an electrosorption filter for removing toxins, small and middle-sized molecules from the patient blood plasma.

9. Device according to claims 7 and 8, **characterized in that** said hemofilter and said electrosorption filter are combined to form the filter pad wherein said permeable membrane is formed by said hemofilter.

10. Device according to any one of the preceding claims, **characterized in that** said device further comprises means for supplementing said dialysate fluid and/or said (purified) blood plasma with at least one substance selected from the group consisting of vitamins, minerals, anticoagulants, anti microbial agents and other medicaments.

11. Device according to any one of the preceding claims, **characterized in that** said device further comprises ion exchange systems.

12. Device according to any one of the preceding claims, **characterized in that** the performance of the sorption filter is being monitored with a sensor system measuring the quality of the dialysate, blood or bloodplasma in the outlet.

13. Device according to any one of the preceding claims, **characterized in that** the device comprises a control unit controlling the sorption activity of the sorption material by a voltage between 0-200V of an external power source in order ensure a field strength in the sorption medium of typically 10-20 V/cm.

14. Device according to claims 12 and 13, **characterized in that** the voltage of the external source is electronically regulated depending on the reading of the sensor system.

15. Device according to claim 13 or 14, **characterized in that** the voltage of the external source can be reversed into opposite values in order to repell and to release toxins and to regenerate the sorption materials.

16. Device according to any one of the preceding claims, **characterized in that** the electrodes are planar, circular, tubular or thread electrodes made from Ag, Ti, stainless steel, Cr or other blood compatible materials or with a coating hereof.
